(19) Europäisches Patentamt
European Patent Office
Office européen des brevets

(11) Veröffentlichungsnummer: **0 008 013**
**B1**

(12) # EUROPÄISCHE PATENTSCHRIFT

(45) Veröffentlichungstag der Patentschrift: **17.02.82**

(21) Anmeldenummer: **79102427.6**

(22) Anmeldetag: **13.07.79**

(51) Int. Cl.³: **C 07 C 47/45**, C 07 C 45/29, C 07 C 47/21, C 07 C 49/203, C 07 C 49/403, C 07 C 69/67, C 07 D 307/20

(54) **Verfahren zur Herstellung von Carbonylverbindungen.**

(30) Priorität: **19.07.78 DE 2831595**

(43) Veröffentlichungstag der Anmeldung: **20.02.80 Patentblatt 80/4**

(45) Bekanntmachung des Hinweises auf die Patenterteilung: **17.02.82 Patentblatt 82/7**

(84) Benannte Vertragsstaaten: **BE CH DE FR GB IT NL**

(56) Entgegenhaltungen:
**DE-A1-2 520 219**

**ZEITSCHRIFT FÜR ELEKTROCHEMIE (Berichte der Bunsen-Gesellschaft für physikalische Chemie) 1953 Weinheim/Bergstraße K. SCHWABE et al. »Polarographische Studien an Terpenderivaten. IV. Mitteilung: Aldehyde und deren Homologe« Seiten 293 bis 301**
**Chemical Abstracts, Band 81, 1974 (COLUMBUS, OHIO, USA) YU. S. MARDASHEV et al. »Memory effect for silver catalysts«, Seite 355, Spalte 1, Abstract Nr. 49 203 E**

(73) Patentinhaber: **BASF Aktiengesellschaft, Carl-Bosch-Strasse 38, D-6700 Ludwigshafen (DE)**

(72) Erfinder: **Lehmann, Gunter, Bayernstrasse 58, D-6700 Ludwigshafen (DE)**
Erfinder: **Dudeck, Christian, Dr.-Chem., Odenwaldring 20, D-6703 Limburgerhof (DE)**
Erfinder: **Petri, Norbert, Dr.-Chem., Max-Beckmann-Strasse 17, D-6710 Frankenthal (DE)**
Erfinder: **Meissner, Bernd, Dr.-Chem., Dammweg 15, D-6900 Heidelberg 1 (DE)**
Erfinder: **Fliege, Werner, Dr.-Chem., c/o BASF Wyandotte Corp.,Central R+D P.O.Box 111, Wyandotte, Mich. 48192 (US)**
Erfinder: **Sauer, Wolfgang, Dr.-Chem., Kaefertaler Strasse 183, D-6800 Mannheim 1 (DE)**
Erfinder: **Ross, Karl-Heinz, Dr.-Chem., Sudetenstrasse 8, D-6704 Mutterstadt (DE)**
Erfinder: **Halbritter, Klaus, Dr.-Chem., Schwarzwaldstrasse 19, D-6800 Mannheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von Carbonylverbindungen

Die Erfindung betrifft ein neues Verfahren zur Herstellung von Carbonylverbindungen durch Oxidation von Alkoholen in Gegenwart eines Silberkatalysators und/oder Kupferkatalysators bestimmter Korngröße und unter bestimmten Bedingungen der Temperatur und Verweilzeit.

Es ist aus der US-Patentschrift 2 042 220 bekannt, daß man 3-Methyl-3-buten-1-ol mit Sauerstoff im Überschuß bei 360 bis 550°C in Gegenwart von Metallkatalysatoren, z. B. Kupfer- und Silberkatalysatoren, zu 3-Methyl-3-buten-1-al oxidiert. Die Katalysatoren können Legierungen, Metallverbindungen oder elementares Metall sein. Bevorzugt sind aktivierte Katalysatoren; als Aktivierungsoperationen werden eine Oberflächenamalgamierung des Metalls und anschließendes Erhitzen der Metalloberfläche angegeben. Die Herstellung von Kupfer- und Silberkatalysatoren in den Beispielen besteht aus der Reduktion von Kupferoxiddraht bei 300°C in einer Wasserstoffatmosphäre oder Amalgamierung und Erhitzen von Silberdrahtnetzen. Wie die deutsche Offenlegungsschrift 2 041 976 lehrt, entstehen bei der Umsetzung erhebliche Mengen von Isovaleraldehyd und Isopren. Bei Zumischung von Sauerstoff erhält man zunehmende Mengen von Dimethylacrylsäure und wesentliche Ausbeuteverluste. Die deutsche Offenlegungsschrift 2 517 859 weist nach, daß als Endstoff nicht das 3-Butenal sondern tatsächlich 3-Methyl-2-buten-1-al erhalten wird.

Die deutsche Offenlegungsschrift 2 517 859 beschreibt die Dehydrierung ungesättigter Alkohole an einem Kupferkatalysator mit einer spezifischen Oberfläche von 0,01 bis 1,5 $m^2/g$ im wesentlichen in Abwesenheit von Sauerstoff bei 150 bis 300°C. Im Falle von $\alpha,\beta$-ungesättigten Alkoholen als Ausgangsstoffe werden gesättigte Aldehyde als Nebenprodukte gebildet; die Selektivität für $\alpha,\beta$-ungesättigte Aldehyde ist gering (Seite 2, letzter Absatz). Im Hinblick auf den Stand der Technik, dem zum Zeitpunkt der Anmeldung die vorgenannten Veröffentlichungen schon angehörten, lehrt die Druckschrift, daß nach den üblichen Verfahren aus $\beta,\gamma$-ungesättigten Alkoholen nur sehr schwierig $\beta,\gamma$-ungesättigte Aldehyde zu erhalten sind, da hohe Anteile an gesättigten Aldehyden und $\alpha,\beta$-ungesättigten Aldehyden entstehen (Seite 3, 1. Absatz). Solche Gemische der $\alpha,\beta$- und $\beta,\gamma$-ungesättigten Aldehyde müssen dann ihrerseits, z. B. nach dem in DBP 2 243 810 beschriebenen Verfahren, vom unumgesetzten Alkohol getrennt werden. Bei der in der deutschen Offenlegungsschrift 1 517 859 beschriebenen Arbeitsweise muß die Konzentration an Sauerstoff auf das höchstens 1/10 molarfache des Ausgangsalkohols eingestellt werden und ist vorzugsweise so gering wie möglich.

Die deutsche Patentschrift 2 020 865 lehrt, daß $\beta,\gamma$-ungesättigte Alkohole bei Temperaturen von 150 bis 600°C in Gegenwart von Mischkatalysatoren, z. B. von Silber oder Kupfer und Metalloxiden, zu $\alpha,\beta$-ungesättigten Aldehyden dehydriert werden. In den Beispielen werden bei Verwendung von Silber bzw. Kupfer stets ein Mischkatalysator dieser Metalle mit MgO oder ZnO aufgeführt. Die deutsche Patentschrift 2 243 810 weist darauf hin, daß gute Ausbeuten mit dieser Arbeitsweise nur erhalten werden, wenn der Ausgangsstoff unvollständig umgesetzt wird. Ebenfalls ist die destillative Trennung des Endstoffs vom unumgesetzten Ausgangsstoff durch einfache Destillation wegen des geringen Siedepunktunterschiedes unmöglich. Die Reindarstellung ist somit aufwendig und mit großen Ausbeuteverlusten verbunden.

Aus der deutschen Offenlegungsschrift 2 041 976 ist es bekannt, daß man 3-Methyl-2-buten-1-ol bei 150 bis 600°C in Gegenwart von Dehydrierungskatalysatoren und Zusatzstoffen wie basischen Metalloxiden, Stickstoff-, Phosphor- oder Schwefelverbindungen zu 3-Methyl-2-buten-1-al umsetzt. Als Katalysatoren werden in den Beispielen Messingspäne, Kupferoxid/Zinkoxid/Chromoxid/Aluminiumoxid, Kupfer/Zinkoxid, Silber/Magnesiumoxid, Silber/Zinkoxid genannt. Die deutsche Offenlegungsschrift 2 041 976 lehrt (Seite 3), daß den reinen Metallkatalysatoren im allgemeinen oxidische Katalysatoren überlegen sind und erhebliche Verbesserungen sich bei der Dehydrierung ergeben, wenn der eigentliche Dehydrierungskatalysator basische Metalloxide enthält. Außerdem sollen organische Verbindungen mit nukleophilen Eigenschaften, u. a. Schwefelverbindungen, Nebenreaktionen unterdrücken (Seite 4 unten). Nachteilig ist, daß solche Verbindungen mit nukleophilen Eigenschaften, z. B. Schwefelverbindungen, teilweise Katalysatorgifte sind. Zusätzliche Nachteile sind die Zumischung von Inertgasen, z. B. 20 bis 50 Volumenprozent Wasserdampf, und die im Reaktionsaustrag enthaltenen, bei der Reaktion zugesetzten Verbindungen mit nukleophilen Eigenschaften.

Die DRP 447 838 beschreibt ein Verfahren, worin Oxosäureester aus den entsprechenden Oxysäureestern durch Umsetzung mit Luft in der Gasphase an Katalysatoren hergestellt werden. Die Katalysatoren sind Oxide von saurem Charakter, die sich von Elementen mit mehreren Oxidationsstufen ableiten, oder Metallsalze der sich von diesen Oxiden ableitenden Säuren. Der Katalysator kann auch auf Trägern wie Kupfer, aufgebracht werden. Die Reaktionstemperatur beträgt 200 bis 400°C. Ausbeuten von 60 bis 70 Prozent der Theorie werden beschrieben. Im Falle der Oxidation von Milchsäurealkylestern zeigen die Beispiele nur Vanadiumpentoxid als Katalysator. Silbervanadat wird nur für die Oxidation von Glykolsäureäthylester verwendet. Nachteilig ist, daß statt mit Luft mit Sauerstoff im Überschuß oxidiert wird und Reaktionsrohre von 10 Meter Länge verwendet werden müssen.

In Beispiel 4 wird beschrieben, daß das Endprodukt noch wenige Prozente unveränderten

Milchsäureester enthält. Nun ist aber aus Can. J. Res., Band 24, Seite 221 (1946) bekannt, daß Mischungen aus Milchsäureäthylester und Brenztraubensäureäthylester durch fraktionierte Destillation schwierig zu trennen sind, so daß die Gewinnung der Ester in reiner Form bei diesem Verfahren nur durch hohen technischen Aufwand zu erreichen ist.

Um die schwierige Trennung von Endprodukt und Ausgangsprodukt zu verhindern, benötigt man nach Can. J. Res. (loc. cit), Seiten 221 bis 223, einen Katalysator, der bei der Umsetzung von Milchsäureäthylester zu Brenztraubensäureäthylester vollständigen Umsatz bewirkt. An einem mit Platinmohr elektrolytisch beschichteten Platinnetz wird bei gutem Umsatz eine Ausbeute von 68 Prozent erreicht. Der Katalysator ist unwirtschaftlich, aufwendig in der Regenerierung und hat nur eine Lebensdauer von ca. 45 Stunden. Die Raum-Zeit-Ausbeute beträgt nur 0,085 Gramm pro Kubikzentimeter Katalysatorvolumen und Stunde. Es wird darauf hingewiesen, daß verschiedene Metallkatalysatoren, darunter auch Silber auf Kupferdrahtnetz, in ihrer Wirkung ungenügend sind; bei 250 bis 300° C erreicht man mit ihnen nur 35 bis 50 Prozent Umsatz.

Im Hinblick auf den Stand der Technik weist Ullmanns Encyklopädie der technischen Chemie, Band 4, Seite 727, darauf hin, daß trotz verschiedener synthetischer Bildungsmöglichkeiten das Erhitzen von Weinsäure mit Kaliumbisulfat die beste Darstellung von Brenztraubensäure bleibt. Eine Ausbeute von 50 bis 55 Prozent der Theorie wird bei diesen Verfahren beschrieben.

Es ist aus den Chemical Abstracts, Vol. 84 (1976), Seite 440 (5153 w) bekannt, daß eine Dehydrierung von 4-tert.-Butylcyclohexanol in Gegenwart eines Kupferkatalysators bei 125 bis 220°C durchgeführt wird. Da nur die Ausbeute von zwei Syntheseschritten mit 70 bis 75 Prozent angegeben ist, kann für den Dehydrierschritt nur eine Ausbeute unter 75 Prozent angenommen werden. Nachteilig bei diesem Verfahren ist, daß der Mischkatalysator nur schwierig aufgearbeitet werden kann.

Die deutsche Offenlegungsschrift 1 618 102 beschreibt eine Dehydrierung von gegebenenfalls alkylsubstituierten Cycloalkanolen zwischen 300 bis 450°C in der Gasphase. Verwendet wird ein Mischkatalysator, der mehr als 90 Gewichtsprozent Zinkoxid, bis 3 Gewichtsprozent Silber oder Cadmium, bis 3 Gewichtsprozent wenigstens eines der Elemente Vanadium, Molybdän, Wolfram oder Mangan und bis 3 Gewichtsprozent Kupfer enthält. Beispiele 2 und 5 zeigen für die Umsetzung von Methylcyclohexanol bzw. Cyclohexanol Ausbeuten von 93 Prozent bzw. 94,6 Prozent.

Es wurde nun gefunden, daß man Carbonylverbindungen der Formel I

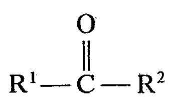

(I)

worin $R^1$ ein Wasserstoffatom oder einen aliphatischen Rest mit mindestens 2 Kohlenstoffatomen oder einen cycloaliphatisch-aliphatischen Rest bedeutet, $R^2$ den Rest

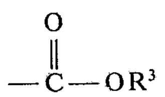

bezeichnet, $R^3$ für einen aliphatischen, araliphatischen, cycloaliphatischen oder aromatischen Rest steht,

$R^2$ darüber hinaus auch ein Wasserstoffatom bedeuten kann, wenn $R^1$ einen cycloaliphatisch-aliphatischen Rest oder einen ungesättigten aliphatischen Rest, der, sofern er nur eine Doppelbindung und diese Doppelbindung in $\alpha,\beta$-Stellung oder in $\beta,\gamma$-Stellung zum mit $R^1$ und $R^2$ benachbarten Kohlenstoffatom trägt, in $\beta$-Stellung unverzweigt ist oder dessen Kohlenstoffatom in $\gamma$-Stellung noch mit einem Heteroatom oder mit mindestens einem Kohlenstoffatom in $\delta$-Stellung verbunden ist, bezeichnet,

$R^1$ und $R^2$ zusammen mit dem benachbarten Kohlenstoffatom auch für Glieder eines alicyclischen Restes oder eines cyclischen Äthers stehen, durch Oxidation von Alkoholen mit Sauerstoff in Gegenwart eines Metallkatalysators vorteilhaft erhält, wenn man Alkohole der Formel II

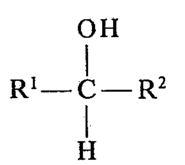

(II)

worin $R^1$ und $R^2$ die vorgenannte Bedeutung besitzen, in Gegenwart von Silberkristallen und/oder Kupferkristallen mit einer Korngröße von 0,01 Mikrometer bis 2,5 Millimeter als Katalysator bei einer Temperatur von 450 bis 700° C während einer Verweilzeit von höchstens 0,1 Sekunden oxidiert.

Die Umsetzung kann für den Fall der Verwendung von Glykolsäuremethylester oder

4-tert.-Butylcyclohexanol durch die folgenden Formeln beschrieben werden:

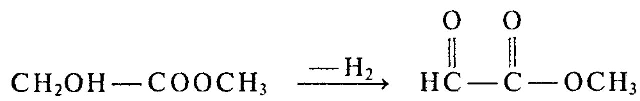

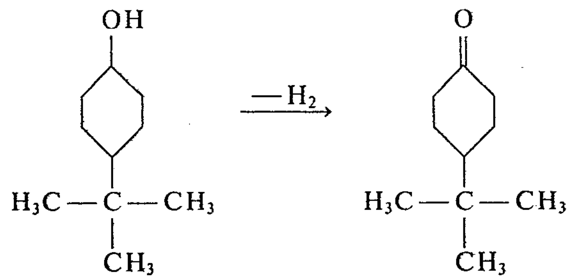

Im Vergleich zum Stand der Technik liefert das Verfahren nach der Erfindung überraschend auf teilweise einfacherem und wirtschaftlicherem Wege ein besseres Gesamtergebnis mit Bezug auf Ausbeute, Raum-Zeit-Ausbeute und Reinheit des Endstoffs sowie Lebensdauer des Katalysators. Kostspielige Katalysatoraufbereitung bzw. aufwendige Reaktoren werden vermieden. Der Katalysator ist vergleichsweise leicht regenerierbar. Silberkristalle aller Teilchengrößen, wie die auch bei der elektrolytischen Herstellung des Silbergranulats anfallen, werden verwendet. Bei dem erfindungsgemäßen Verfahren werden somit die Elektrolysieranlagen besser ausgenutzt und können entsprechend dimensioniert werden; Energie, Betriebspersonal und Hilfsstoffe, z. B. Salpetersäure, werden eingespart und Operationen wie Wäsche, Siebung und Trocknung des Silbers vereinfacht. Alle diese vorteilhaften Ergebnisse sind im Hinblick auf den Stand der Technik überraschend. Es war nach der Lehre der deutschen Offenlegungsschrift 2 020 865 zu erwarten, daß bei der Dehydrierung von $\beta,\gamma$-ungesättigten Alkoholen am reinen Silberkatalysator keine $\beta,\gamma$-ungesättigten, sondern $\alpha,\beta$-ungesättigte Aldehyde anfallen. Metallisches Silber bei der Dehydrierung von ungesättigten Alkoholen erscheint auch nach den Angaben in DE-OS 2 041 976 als ungeeignet. Die erfindungsgemäßen Ergebnisse sind ebenfalls im Hinblick auf DE-OS 2 517 859 überraschend, da diese lehrt, daß die Reaktion im wesentlichen in Abwesenheit von Sauerstoff und bei einer Reaktionstemperatur von 150 bis 300°C mit Dampf als Trägergas durchzuführen ist; außerdem muß der Katalysator reaktiviert werden (loc. cit., Seite 7, dritter Abschnitt). Eine Reaktivierung des erfindungsgemäßen Katalysators ist auch bei mehr als 1000 Betriebsstunden nicht nötig. Im Hinblick auf die Lehre in Can. J. Res. (loc. cit.) hätte man angesichts der hohen erfindungsgemäßen Temperaturen und des erfindungsgemäßen Katalysators zumindest eine wesentliche Verschlechterung der Ausbeute und erhebliche Bildung von Zersetzungsprodukten erwarten sollen. Im Vergleich zu dem in der deutschen Offenlegungsschrift 1 618 102 beschriebenen Verfahren ist insbesondere der Durchsatz an Ausgangsstoff höher. Es ist weiterhin überraschend, daß bei den hohen Reaktionstemperaturen an Silber als Katalysator auch mit verzweigten Alkylgruppen substituierte Cyclohexanone in hohen Ausbeuten und Raum-Zeit-Ausbeuten hergestellt werden können. Im Hinblick auf den Stand der Technik waren Bildung von alkylsubstituierten Phenolen, Cyclohexen oder von Kondensationsprodukten wie Cyclohexylcyclohexenol oder Cyclohexylcyclohexanon zu erwarten.

Bevorzugte Ausgangsstoffe II und IIa und dementsprechend bevorzugte Endstoffe I und Ia sind solche, in deren Formeln $R^1$ ein Wasserstoffatom oder einen Alkylrest oder Alkenylrest mit 2 bis 18, insbesondere 2 bis 8 Kohlenstoffatomen oder darüber hinaus einen Alkylrest mit 1 bis 18, insbesondere 1 bis 5 Kohlenstoffatomen oder einen Alkenylrest mit 2 bis 18, insbesondere 2 bis 5 Kohlenstoffatomen, die jeweils einen bicyclischen oder monocyclischen Cycloalkylrest oder Cycloalkenylrest mit 5 bis 12 Kohlenstoffatomen tragen, bedeutet, $R^2$ den Rest

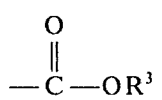

bezeichnet, $R^3$ für einen Alkylrest mit 1 bis 18, insbesondere 1 bis 5 Kohlenstoffatomen, einen Cycloalkylrest mit 5 bis 12 Kohlenstoffatomen, einen Aralkylrest oder Alkylarylrest mit 7 bis 12 Kohlenstoffatomen, oder einen Phenylrest steht, $R^2$ darüber hinaus auch ein Wasserstoffatom bedeuten kann, wenn $R^1$ einen cycloaliphatisch-aliphatischen Rest oder einen ungesättigten aliphatischen Rest, der, sofern er nur eine Doppelbindung und diese Doppelbindung in $\alpha,\beta$-Stellung oder $\beta,\gamma$-Stellung zum mit $R^1$ und $R^2$ benachbarten Kohlenstoffatom trägt, in $\beta$-Stellung unverzweigt ist oder dessen Kohlenstoffatom in $\gamma$-Stellung noch mit einem Heteroatom oder mit mindestens einem Kohlenstoffatom in $\delta$-Stellung verbunden ist, bezeichnet, $R^1$ und $R^2$ zusammen mit dem benachbarten

Kohlenstoffatom auch für Glieder eines alicyclischen Ringes mit 5 oder 6 Kohlenstoffatomen, der vorteilhaft 1 bis 3 Alkylgruppen, vorzugsweise verzweigte, insbesondere tertiäre Alkylgruppen mit jeweils 1 bis 10, insbesondere 1 bis 7 Kohlenstoffatomen je geradkettige Alkylgruppe oder 3 bis 10 Kohlenstoffatomen je verzweigte Alkylgruppe oder 4 bis 10 Kohlenstoffatomen je tertiäre Alkylgruppe trägt, oder auch für Glieder eines cyclischen Äthers mit 5 oder 6 Kohlenstoffatomen stehen, $R^4$ in $\beta,\gamma$-Stellung eine Doppelbindung trägt und einen Alkenylrest mit 3 bis 18, insbesondere 3 bis 8 Kohlenstoffatomen, der, sofern er nur eine Doppelbindung und diese am Ende einer Kohlenstoffkette trägt, in $\beta$-Stellung unverzweigt ist, oder darüber hinaus einen Alkylrest mit 1 bis 8, insbesondere 1 bis 5 Kohlenstoffatomen oder Alkenylrest mit 2 bis 18, insbesondere 2 bis 5 Kohlenstoffatomen, die jeweils einen bicyclischen oder monocyclischen Cycloalkylrest oder Cycloalkenylrest mit 5 bis 12 Kohlenstoffatomen tragen, bedeutet, $R^5$ die Bedeutung von $R^4$ besitzt, an Stelle der Doppelbindungen in $\beta,\gamma$-Stellung aber eine Doppelbindung in $\alpha,\beta$-Stellung trägt. Die vorgenannten Reste können noch durch unter den Reaktionsbedingungen inerte Gruppen, z. B. Alkylgruppen mit jeweils 1 bis 4 Kohlenstoffatomen, substituiert sein.

Als geeignete Ausgangsstoffe II bzw. IIa kommen z. B. in Frage: Der Methyl-, Äthyl-, Propyl-, Isopropyl-, Butyl-, Isobutyl-, sek.-Butyl-, tert.-Butyl-, Cyclohexyl-, Cyclopentyl-, Benzyl-, Phenylester der Glykolsäure, 2-Hydroxybuttersäure, 2-Hydroxyvaleriansäure, 2-Hydroxycapronsäure, 2-Hydroxyönanthsäure, 2-Hydroxycaprylsäure, 2-Hydroxypelargonsäure, 2-Hydroxycaprinsäure; But-3,4-enyl-, Pent-3,4-enyl-, Hex-3,4-enyl-, Hept-3,4-enyl-, Oct-3,4-enyl-alkohol, Pent-4,5-enyl-, Hex-4,5-enyl-, Hept-4,5-enyl-, Oct-4,5-enyl-alkohol, Hex-5,6-enyl-, Hept-5,6-enyl-, Oct-5,6-enyl-alkohol sowie die homologen in 2-, 3-, 4- oder 5-Stellung durch die Methyl-, Äthyl-, Propyl-, Isopropyl-, Butyl-, Isobutyl-, sek.-Butyl-, tert.-Butyl-gruppe substituierte Alkenole; 4-Penten-2-ol, 3-Cyclohexyl-but-3,4-enyl-alkohol, 1-(2'-Hydroxyäthyl)-cyclohexen, Isohomoperillaalkohol der Formel

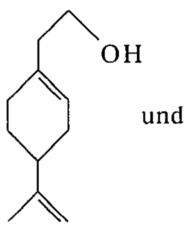

und Homoperillaalkohol der Formel

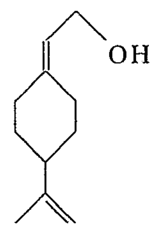

Nopol der Formel

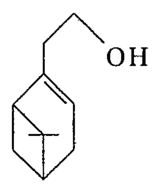

Cyclohexanol und entsprechende durch die Methyl-, Äthyl-, Isopropyl-, Butyl-, Isobutyl-, sek.-Butyl-, Pentyl-, Hexyl- und insbesondere tert.-Butyl-, tert.-Pentyl-, tert.-Hexyl-, tert.-Heptyl-, tert.-Octyl-, tert.-Nonyl-, tert.-Decylgruppe einfach, zweifach oder dreifach substituierte Cyclohexanole; 3-Hydroxytetrahydrofuran, 3-Hydroxy-tetrahydropyran, 4-Hydroxytetrahydropyran.

Bei den $\beta,\gamma$-ungesättigten cycloaliphatisch-aliphatischen Resten kann die Doppelbindung in $\beta,\gamma$-Stellung sowie gegebenenfalls andere Doppelbindung sowohl im cycloaliphatischen Ring wie auch in der aliphatischen Kette liegen.

Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Ausgangsstoff II werden zweckmäßig im Molverhältnis von 0,25 bis 0,9, insbesondere von 0,35 bis 0,7 Mol Sauerstoff je Mol Ausgangsstoff II angewandt. Man verwendet gegebenenfalls unter den Reaktionsbedingungen inerte Lösungsmittel, z. B. Wasser oder Äther wie Tetrahydrofuran und Dioxan; Aceton oder entsprechende Gemische. Zweckmäßig wird für die Reaktion ein Verhältnis von 10 bis 1000, vorzugsweise 100 bis 300 Gewichtsprozent Lösungsmittel je Gewichtsmenge Ausgangsstoff II gewählt. Der Ausgangsstoff II wird in Dampfform, gegebenenfalls im Gemisch mit Wasserdampf bzw. Lösungsmitteldampf und gegebenenfalls mit Inertgas, dem Reaktionsraum zugeführt. Die Verweilzeit im Reaktionsraum beträgt zweckmäßig 0,0005 bis 0,1, vorteilhaft von 0,0005 bis 0,04, insbesondere von 0,001 bis 0,03, bevorzugt 0,001 bis 0,021 Sekunden. Die Verweilzeit wird auf die Reaktionszone ohne Katalysatorfüllung bezogen und so berechnet. Als Berechnungsgrundlage kann z. B. der Reaktionsraum eines leeren Reaktorrohres dienen.

Die Gesamtschichtdicke des Katalysators beträgt zweckmäßig 5 bis 50, vorzugsweise 20 bis 30 Millimeter. Man kann alle Korngrößen von 0,01 Mikrometer bis 2,5 Millimeter, vorzugsweise 0,1 Millimeter bis 2,5 Millimeter, im Katalysator verwenden, wobei man zweckmäßig eine homogene Vermischung aller Korngrößen in der Katalysatorschicht wählt. Ebenfalls kann man den Katalysator zwar mit allen vorgenannten Korngrößen bilden, diese aber je nach Korngröße in 2, 3 oder mehr Schichten anordnen. Verwendet man alle Korngrößen im Katalysator, ist ein 3-Schichtenkatalysator,

2-Schichtenkatalysator oder ein Einschichtenkatalysator bevorzugt. Die Katalysatorteilchen in Gestalt von Silberkristallen und/oder Kupferkristallen befinden sich in Katalysatoren mit mehreren Schichten, z. B. im 2- bzw. 3-Schichtkatalysator des üblicherweise vertikal aufgestellten Reaktors je nach Korngröße in einem oberen und unteren (2-Schichtkatalysator) bzw. oberen, mittleren oder unteren Teil (3-Schichtkatalysator) der Gesamtschicht angeordnet. Das Ausgangsgemisch aus Dampf des Ausgangsstoffs II und Sauerstoff bzw. Luft und gegebenenfalls Wasserdampf und Inertgas wird im allgemeinen von oben nach unten geführt, so daß die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäß alle Angaben der Beschreibung über oberen (unteren) Teil des Katalysators für den entsprechenden, dem Ausgangsgemisch (dem abgeführten Reaktionsgemisch) zugewandten Teil, z. B. bei horizontal angeordneten Reaktoren für den vorderen (hinteren) Teil des Katalysators. Das gesamte Katalysatorbett liegt zweckmäßig auf einem Netz aus Silber oder Edelstahl (vorgeglüht). Bei großen Reaktoren mit einem Durchmesser von mehr als 15 cm wird das Netz zweckmäßig vor Einbau gewellt.

In einer bevorzugten Ausführungsform verwendet man nicht alle Korngrößen, sondern nur den grobkörnigen Anteil von Silberkristallen und/oder Kupferkristallen mit einer Korngröße von 0,1 bis 2,5 Millimeter. Katalysatoren, deren sämtliche Silberkristalle und/oder Kupferkristalle nur diese gröberen Kristalle enthalten, werden im folgenden als grobkörnige Katalysatoren definiert.

Der grobkörnige Anteil (Korngröße von 0,1 bis 2,5 Millimeter) kann in einer Schicht oder in mehreren Schichten angeordnet sein, vorteilhaft in einer Schicht und insbesondere in 2 oder 3 Schichten. Im Einschichtkatalysator wird zweckmäßig der grobkörnige Anteil in homogener Verteilung seiner Katalysatorteilchen durch den ganzen Katalysator hindurch vorliegen. Im 2-Schichtkatalysator haben bevorzugt seine obere Silber- und/oder Kupferschicht einen Anteil von 5 bis 30, vorzugsweise 10 bis 20 Gewichtsprozent und Teilchen einer Korngröße von 0,1 bis 0,75 Millimeter und seine untere Silber- und/oder Kupferschicht einen Anteil von 70 bis 95, vorzugsweise von 80 bis 90 Gewichtsprozent und Teilchen einer Korngröße von 0,75 bis 2,5 Millimeter.

Ist der grobkörnige Anteil in 3 Schichten angeordnet, so befinden sich vorteilhaft im unteren Teil 72,5 bis 89, vorzugsweise 77,5 bis 82,5 Gewichtsprozent aller Katalysatorteilchen, im mittleren Teil 2,5 bis 7,5, vorzugsweise 4,5 bis 6,5 Gewichtsprozent aller Katalysatorteilchen, im oberen Teil 8,5 bis 20, vorzugsweise 13 bis 16 Gewichtsprozent aller Katalysatorteilchen. Die Teilchen des unteren Schichtteils haben vorteilhaft Korngrößen von 1 bis 2,5, die des mittleren Schichtteils von 0,75 bis 1, die des oberen Schichtteils 0,1 bis 0,75 Millimeter. Jeder Schichtteil (grobkörniger Anteil) kann aus einer oder mehreren Schichten, vorzugsweise aus 1 oder 2 Schichten bestehen. Bevorzugt ist ein 2- bis 4-Schichtenkatalysator (grobkörnig). Jede dieser Schichten unterscheidet sich von der anderen in der Korngröße der Silberkristalle und/oder Kupferkristalle und meistens auch im zugehörigen Gewichtsanteil des Gesamtkatalysators (grobkörnig).

Hat der obere Schichtteil 2 Schichten, so haben seine untere Schicht bevorzugt einen Anteil von 0,5 bis 2 Gewichtsprozent und Teilchen einer Korngröße von 0,4 bis 0,75 Millimeter und seine obere Schicht entsprechend einen Gewichtsanteil von 8 bis 18 Gewichtsprozent und Teilchen der Korngröße von 0,1 bis 0,4 Millimeter. Entsprechend sind bei dem mittleren Schichtteil mit Bezug auf Gewichtsanteil (Korngröße der Teilchen) bevorzugt:

2 Schichten: obere Schicht 1,5 bis 4,5 (0,75 bis 0,9 mm) Gew.-%;
untere Schicht 1 bis 3 (0,9 bis 1 mm) Gew.-%.

Bei dem unteren Schichtteil sind bevorzugt:

2 Schichten: obere Schicht 7,5 bis 22,5 (1 bis 1,75 mm) Gew.-%
untere Schicht 50 bis 81,5 (1,75 bis 2,5 mm) Gew.-%

Das feinkörnige Silber und/oder Kupfer (Korngröße 0,01 bis 10 µm) kann nach bekannten Methoden, z. B. bei der Silber- und/oder Kupfergewinnung mit entsprechenden Mahloperationen und Sieboperationen oder in Gestalt von Raney-Silber hergestellt werden. Bezüglich der Herstellungsmethoden von Silber wird auf Ullmanns Encyklopädie der technischen Chemie, Band 15, Seiten 636 bis 666, verwiesen. Ebenfalls kann Silber aus entsprechenden Lösungen, z. B. Silbernitratlösungen mit Fällungsmitteln, z. B. mit Hydrazin oder Formaldehyd, ausgefällt oder durch Elektrolyse gewonnen werden.

Ein besonders vorteilhafter Katalysator hat die folgende Zusammensetzung:

| | | |
|---|---|---|
| Schicht 1 (oberste): | 8—18 Gew.-% | des Katalysators mit Teilchen der Korngröße 0,1—0,4 mm |
| Schicht 2: | 0,5—2 Gew.-% | des Katalysators mit Teilchen der Korngröße 0,4—0,75 mm |
| Schicht 3: | 2,5—7,5 Gew.-% | des Katalysators mit Teilchen der Korngröße 0,75 —1 mm |

| | | |
|---|---|---|
| Schicht 4 (unterste): | 72,5—89 Gew.-% | des Katalysators mit Teilchen der Korngröße 1—2,5 mm. |

Bevorzugt sind in allen diesen Katalysatoranordnungen Gemische von Silber und Kupfer oder insbesondere Silberkristalle allein.

Zweckmäßig belastet man den Katalysator mit 0,5 bis 10 t, insbesondere 0,7 bis 6 t Ausgangsstoff II je $m^2$ Katalysatorbettquersachnitt und Stunde. Zur großtechnischen Ausführung verwendet man bevorzugt Katalysatorbettdurchmesser von mindestens 0,1, zweckmäßig 0,2 bis 3 Meter.

Die Oxidation kann wie folgt durchgeführt werden: In ein Verdampfungsaggregat, z. B. einen Fallstromverdampfer, werden einzeln oder im Gemisch Ausgangsstoff II und gegebenenfalls Wasser oder Lösungsmittel eingegeben und verdampft. Dann leitet man das Gasgemisch aus dampfförmigem Ausgangsstoff II, Luft, gegebenenfalls Inertgas und gegebenenfalls Wasserdampf oder Lösungsmitteldampf in vorgenannten Mengen bei Temperaturen von 450 bis 700° C, insbesondere 525 bis 625° C, durch den Katalysator. Die Temperatur wird zweckmäßig im Katalysator durch Thermoelemente gemessen. Ab Beginn der Reaktion leitet man im allgemeinen die Luft kontinuierlich dem dampfförmigen Ausgangsgemisch zu, gegebenenfalls unter Einleiten durch den Sumpf des Verdampfungsaggregats. Das Verfahren wird im allgemeinen bei Drücken zwischen 0,5 und 2 bar, vorzugsweise zwischen 0,8 und 1,8 bar, kontinuierlich durchgeführt. Es ist dabei vorteilhaft, die die Katalysatorzone verlassenden Reaktionsgase innerhalb kurzer Zeit abzukühlen, z. B. auf Temperaturen von 20°C bis 160°C. Der Hauptteil des Endstoffs I wird häufig so kondensiert. Das abgekühlte Gasgemisch wird dann zweckmäßig einem Absorptionsturm zugeführt, in welchem der Endstoff I mit einem geeigneten Lösungsmittel wie Dimethylformamid, Dimethylsulfoxid, Aceton, Methanol oder Wasser sowie deren Gemische und/oder in vorgelegtem Kondensat früherer Umsetzungen, vorteilhaft im Gegenstrom, aus dem Gasgemisch gewaschen wird. Aus Kondensat und den Absorbaten wird der Endstoff I dann in üblicher Weise, z. B. durch Destillation, isoliert.

Die nach dem Verfahren der Erfindung herstellbaren Carbonylverbindungen I sind wertvolle Ausgangsstoffe für die Herstellung von Arzneimitteln, Kunstharzen und Kunststoffen. So können z. B. die Ester zu den entsprechenden Carbonsäuren, z. B. mit Wasser in Gegenwart eines Kationenaustauschers bei 80 bis 100° C, verseift werden.

Die nach dem Verfahren der Erfindung herstellbaren, in 2,3- oder 3,4-Stellung ungesättigten Alken-1-ale sind wertvolle Ausgangsstoffe für die Herstellung von Farbstoffen, Schädlingsbekämpfungsmitteln, Pharmazeutika, Kunststoffen, Naturstoffen, Riechstoffen, z. B. Citral, Vitaminen, z. B. Vitaminen A und E, Chrysanthemumsäure. Bezüglich der Verwendung wird auf die deutsche Patentschrift 2 243 810, die deutsche Offenlegungsschrift 2 041 976, deutsche Patentschrift 2 020 865, US-Patentschrift 2 042 220 verwiesen. Bisher konnten aus $\alpha,\beta$- bzw. $\beta,\gamma$-ungesättigten Alkoholen nur in unbefriedigender Weise mit Bezug auf Ausbeute, Reinheit des Endstoffs und einfachen, wirtschaftlichen Betrieb $\alpha,\beta$-ungesättigte Aldehyde hergestellt werden. Die Herstellung von 2-Alkylen-1-olen ist aufwendig; hingegen sind die entsprechenden 3-Alkylenverbindungen leichter zugänglich. Durch die Oxidation solcher 3-Alkylen-1-ole zu 3-Alkylen-1-alen und anschließende Isomerisierung der Reaktionsgemische ergibt sich somit ein einfacher und witschaftlicher Weg, 2-Alkylen-1-ale, Zwischenprodukte für die Vitaminsynthese, in besserer Ausbeute und Reinheit herzustellen.

Die in den folgenden Beispielen angeführten Teile bedeuten Gewichtsteile.

## Beispiel 1

Man verwendet eine Anlage mit Verdampfer und einem senkrechten Rohrreaktor. Der Reaktor enthält an seinem Kopf die Zuführung für das dampfförmige Ausgangsgemisch und die Reaktorhaube. Die Katalysatorschicht liegt unterhalb des Reaktorkopfes, weiter unten folgt eine Kühlzone. Der Reaktor ist mit einer Absorptionskolonne verbunden.

In den Reaktor wird ein Katalysator aus Silberkristallen (14 Teilen) folgender Zusammensetzung eingetragen (Schichthöhe 10 mm):

| | Anteil am Katalysator (Gew.%) | Korngröße (mm) |
|---|---|---|
| Schicht 1 | 14,1 | 0,1 bis 0,75 |
| Schicht 2 | 5,9 | 0,75 bis 1 |
| Schicht 3 | 80,0 | 1 bis 2,5 |

Dem Verdampfer wird pro Stunde ein Gemisch von 1580 Teilen Cyclohexanol und 1378 Teile Luft zugeführt und verdampft. Das dampfförmige Ausgangsgemisch wird durch den Katalysator geleitet und bei 600° C und 1,12 bar umgesetzt. Die Belastung beträgt 5 $t/m^2 \cdot h$. Das gasförmige Reaktionsgemisch wird nun auf 20° C abgekühlt, anschließend mit Wasser gewaschen und fraktioniert destilliert. Man erhält 1363 Teile Cyclohexanon vom Kp. 155° C, entsprechend einer Ausbeute von 88% der Theorie, und 31,2 Teile pro Stunde unumgesetzter Ausgangsstoff II. Die Raum-Zeit-Ausbeute beträgt 434 $g/cm^3 \cdot h$. Die Lebensdauer des Katalysators beträgt 123 Tage, der Umsatz beträgt 98,03 Prozent.

Beispiel 2

Hier wird die gleiche Anlage wie in Beispiel 1 verwendet. In den Reaktor wird ein Katalysator aus Silberkristallen (14 Teilen) folgender Zusammensetzung eingetragen:

| | Anteil am Katalysator (Gew.%) | Korngröße (mm) |
|---|---|---|
| Schicht 1 | 18,5 | 0,1 bis 0,75 |
| Schicht 2 | 81,5 | 0,75 bis 2,5 |

Dem Verdampfer wird pro Stunde ein Gemisch von 235 Teilen 4-tert.-Butylcyclohexanol, 169 Teilen Stickstoff als Inertgas und 144 Teilen Luft zugeführt und verdampft. Das dampfförmige Ausgangsgemisch wird durch den Katalysator geleitet und bei 550° C und 1,03 bar umgesetzt. Die Belastung beträgt 0,75 $t/m^2 \cdot h$. Das gasförmige Reaktionsgemisch wird nun auf 20° C abgekühlt, mit Dioxan gewaschen und fraktioniert destilliert. Man erhält 222,2 Teile pro Stunde 4-tert.-Butylcyclohexanon vom Kp (13 mbar) 96° C, entsprechend einer Ausbeute von 95,8% der Theorie, und 5,8 Teile pro Stunde unumgesetzter Ausgangsstoff II. Die Raum-Zeit-Ausbeute beträgt 71 $g/cm^3 \cdot h$. Die Lebensdauer des Katalysators beträgt 130 Tage, der Umsatz beträgt 97,5 Prozent.

Beispiel 3

Analog Beispiel 2 wird die Umsetzung mit demselben Katalysator, aber mit einer Korngröße von 0,01 Mikrometer bis 0,75 Millimeter in der oberen Silberschicht, durchgeführt. Man erhält dieselben Ergebnisse.

Beispiel 4

Analog Beispiel 2 wird die Umsetzung mit 236 Teilen $\alpha$-Hydroxyisocapronsäure-isobutylester als Ausgangsstoff II bei einer Katalysatortemperatur von 500° C durchgeführt. Die Belastung beträgt 0,76 $t/m^2 \cdot h$. Man erhält 189,1 Teile $\alpha$-Oxoisocapronsäure-isobutylester von Kp 83° C (5 mbar) pro Stunde, entsprechend einer Ausbeute von 81% der Theorie, und 37,8 Teile pro Stunde unumgesetzten Ausgangsstoff II. Die Lebensdauer des Katalysators beträgt 123 Tage, der Umsatz beträgt 84 Prozent. Die Raum-Zeit-Ausbeute beträgt 60 $g/cm^3 \cdot h$.

Beispiel 5

Analog Beispiel 1 wird die Umsetzung mit 410 Teilen pro Stunde 3-Hydroxy-tetrahydrofuran als Ausgangsstoff durchgeführt. Die Belastung beträgt 1,3 $t/m^2 \cdot h$, die Reaktionstemperatur 600° C. Man erhält 308 Teile Tetrahydro-3-furanon vom Kp 140° C pro Stunde, entsprechend einer Ausbeute von 77% der Theorie, und 37 Teile pro Stunde unumgesetzten Ausgangsstoff II. Die Lebensdauer des Katalysators beträgt 123 Tage, der Umsatz beträgt 91 Prozent. Die Raum-Zeit-Ausbeute beträgt 98 $g/cm^3 \cdot h$.

Beispiel 6

Analog Beispiel 5 wird die Umsetzung mit einem Katalysator durchgeführt, bei dem die Silberkristalle der Schicht 1 des Katalysators aus Beispiel 5 durch eine entsprechende Kupferschicht ersetzt wurden. Man erhält 289 Teile Tetrahydro-3-furanon vom Kp 140°C pro Stunde, entsprechend einer Ausbeute von 72% der Theorie, und 41 Teile pro Stunde unumgesetzten Ausgangsstoff. Die Lebensdauer des Katalysators beträgt 123 Tage, der Umsatz beträgt 90 Prozent. Die Raum-Zeit-Ausbeute beträgt 92 g/cm³ · h.

Beispiel 7

Analog Beispiel 1 wird die Umsetzung mit 500 Teilen pro Stunde 4-Methyl-3-penten-2-ol als Ausgangsstoff bei 560°C durchgeführt. Die Belastung beträgt 1,6 t/m² · h. Man erhält 352 Teile 4-Methyl-3-penten-2-on vom Kp 130 bis 131°C pro Stunde, entsprechend einer Ausbeute von 72% der Theorie, und 45 Teile pro Stunde unumgesetzten Ausgangsstoff II. Die Lebensdauer des Katalysators beträgt 123 Tage, der Umsatz beträgt 91 Prozent. Die Raum-Zeit-Ausbeute beträgt 113 g/cm³ · h.

Beispiel 8

Analog Beispiel 1 wird die Umsetzung mit 500 Teilen pro Stunde Homoperillaalkohol als Ausgangsstoff bei 550°C durchgeführt. Die Belastung beträgt 1,6 t/m² · h. Man erhält 213 Teile pro Stunde 7-Formyl-p-mentha-1(2),8-dien (Isohomoperillaaldehyd) der Formel

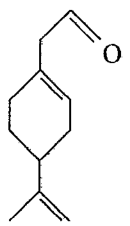

vom Kp 64°C (0,01 mbar) pro Stunde, entsprechend einer Ausbeute von 43% der Theorie sowie 16 Teile pro Stunde 7-Formyl-p-mentha-(1(7),8-dien (Homoperillaaldehyd) der Formel

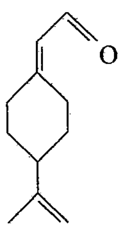

vom Kp 75°C (0,4 mbar), entsprechend einer Ausbeute von 3% der Theorie, und 105 Teile pro Stunde unumgesetzten Ausgangsstoff II. Die Lebensdauer des Katalysators beträgt 123 Tage, der Umsatz beträgt 79 Prozent. Die Raum-Zeit-Ausbeute bezüglich Isohomoperillaaldehyd beträgt 68 g/cm³ · h.

Beispiel 9

Analog Beispiel 1 wird die Umsetzung mit 345 Teilen pro Stunde 3-Penten-1-ol als Ausgangsstoff im Gemisch mit 35 Teilen Wasser bei 550°C durchgeführt. Die Belastung beträgt 1,1 t/m² · h. Man erhält 181 Teile 3-Pentenal vom Kp 106°C pro Stunde, entsprechend einer Ausbeute von 54% der Theorie, sowie 26 Teile 2-Pentenal vom Kp 105°C pro Stunde, entsprechend einer Ausbeute von 8 Prozent, und 71 Teile pro Stunde unumgesetzten Ausgangsstoff II. Die Lebensdauer des Katalysators beträgt 123 Tage, der Umsatz beträgt 79 Prozent. Die Raum-Zeit-Ausbeute bezüglich 3-Pentenal beträgt 58 g/cm³ · h.

Beispiel 10

Analog Beispiel 1 wird die Umsetzung mit 500 Teilen 3-Buten-1-ol als Ausgangsstoff bei 540°C im Gemisch mit 50 Teilen Wasser durchgeführt. Die Belastung beträgt 1,6 t/m$^2$ · h. Man erhält 268 Teile 3-Butenal vom Kp 102 bis 103°C prc Stunde, entsprechend einer Ausbeute von 55% der Theorie, sowie 29 Teile 2-Butenal pro Stunde, entsprechend einer Ausbeute von 6 Prozent, und 122 Teile pro Stunde unumgesetzten Ausgangsstoff II. Die Lebensdauer des Katalysators beträgt 123 Tage, der Umsatz beträgt 75,6 Prozent. Die Raum-Zeit-Ausbeute bezüglich 3-Butenal beträgt 86 g/cm$^3$ · h.

Beispiel 11

Das Reaktionsgemisch (595 Teile) der Umsetzung aus Beispiel 10 (enthaltend 29 Teile 2-Butenal, 145 Teile Wasser, 268 Teile 3-Butenal) wird mit 40 Teilen einer 10gewichtsprozentigen, wäßrigen Lösung von Kaliumacetat versetzt und während 2 Minuten bei 200°C und 25 bar (mit Stickstoff eingestellt) isomerisiert. Man erhält nach Abkühlen und Destillation des Gemisches 264 Teile 2-Butenal vom Kp 101°C (89% der Theorie).

Beispiel 12

Das Reaktionsgemisch (320 Teile) der Umsetzung aus Beispiel 9 (enthaltend 26 Teile 2-Pentenal, 92 Teile Wasser, 181 Teile 3-Pentenal) wird mit 0,5 Teilen Tri-n-butylamin versetzt und während 30 Minuten bei 75°C isomerisiert. Man erhält nach Abkühlen und Destillation des Gemisches 188 Teile 2-Pentenal vom Kp 105°C (91% der Theorie).

Beispiel 13

Ein Reaktionsgemisch aus einer Umsetzung analog Beispiel 8 (enthaltend 16 Teile Homoperillaaldehyd und 213 Teile Isohomoperillaaldehyd) wird mit 0,4 Teilen Tri-n-butylamin versetzt und während 60 Minuten bei 140°C isomerisiert. Man erhält nach Abkühlen des Gemisches und Destillation 147 Teile Homoperillaaldehyd vom Kp 75°C (0,4 mbar) (64% der Theorie).

**Patentanspruch**

Verfahren zur Herstellung von Carbonylverbindungen der Formel I

$$R^1-\overset{\overset{\displaystyle O}{\|}}{C}-R^2 \qquad (I)$$

worin R$^1$ ein Wasserstoffatom oder einen aliphatischen Rest mit mindestens 2 Kohlenstoffatomen oder einen cycloaliphatisch-aliphatischen Rest bedeutet, R$^2$ den Rest

$$-\overset{\overset{\displaystyle O}{\|}}{C}-OR^3$$

bezeichnet, R$^3$ für einen aliphatischen, araliphatischen, cycloaliphatischen oder aromatischen Rest steht,
R$^2$ darüber hinaus auch ein Wasserstoffatom bedeuten kann, wenn R$^1$ einen cycloaliphatisch-aliphatischen Rest oder einen ungesättigten aliphatischen Rest, der, sofern er nur eine Doppelbindung und diese Doppelbindung in $\alpha,\beta$-Stellung oder in $\beta,\gamma$-Stellung zum mit R$^1$ und R$^2$ benachbarten Kohlenstoffatom trägt, in $\beta$-Stellung unverzweigt ist oder dessen Kohlenstoffatom in $\gamma$-Stellung noch mit einem Heteroatom oder mit mindestens einem Kohlenstoffatom in $\delta$-Stellung verbunden ist, bezeichnet,
R$^1$ und R$^2$ zusammen mit dem benachbarten Kohlenstoffatom auch für Glieder eines alicyclischen Restes oder eines cyclischen Äthers stehen, durch Oxidation von Alkoholen mit Sauerstoff in Gegenwart eines Metallkatalysators, dadurch gekennzeichnet, daß man Alkohole der Formel II

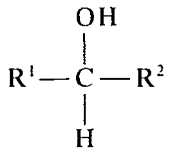

(II)

worin $R^1$ und $R^2$ die vorgenannte Bedeutung besitzen, in Gegenwart von Silberkristallen und/oder Kupferkristallen mit einer Korngröße von 0,01 Mikrometer bis 2,5 Millimeter als Katalysator bei einer Temperatur von 450 bis 700° C während einer Verweilzeit von höchstens 0,1 Sekunden oxidiert.

**Claim**

A process for the preparation of a carbonyl compound of the formula I

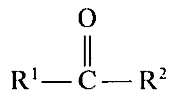

(I)

where $R^1$ is hydrogen or an aliphatic radial of at least 2 carbon atoms or a cycloaliphatic-aliphatic radial, $R^2$ is

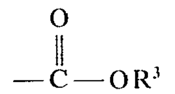

$R^3$ ist an aliphatic, araliphatic, cycloaliphatic or aromatic radical and $R^2$ can also be hydrogen if $R^1$ is a cycloaliphatic-aliphatic radical or an unsaturated aliphatic radical which, if it contains only one double bond and this double bond is in the $\alpha,\beta$-position or in the $\beta,\gamma$-position to the carbon atom adjoining $R^1$ and $R^2$, is unbranched in the $\beta$-position, or which has a carbon atom in the $\gamma$-position bonded to a hetero-atom or to at least one carbon atom in the $\delta$-position, and $R^1$ and $R^2$ together with the adjoining carbon atom may also be members of an alicyclic radical or of a cyclic ether, by oxidizing an alcohol with oxygen in the presence of a metal catalyst, characterized in that an alcohol of the formula II

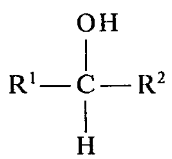

(II)

where $R^1$ and $R^2$ have the above meanings, is oxidized at from 450 to 700° C in the presence of a catalyst comprising silver crystals and/or copper crystals having a particle size of from 0,01 micrometer to 2.5 millimeters, the residence time being at most 0.1 second.

**Revendication**

Procédé pour la préparation de composés carbonylés de formule I

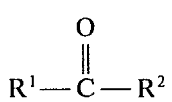

(I)

dans laquelle $R^1$ représente un atome d'hydrogène, un radical aliphatique comportant au moins 2 atomes de carbone ou un radical cycloaliphatique-aliphatique; $R^2$ désigne le radical

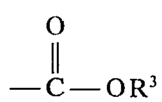

$R^3$ étant mis pour un radical aliphatique, araliphatique, cycloaliphatique ou aromatique; $R^2$ peut en outre représenter un atome d'hydrogène, lorsque $R^1$ désigne un radical cycloaliphatique-aliphatique ou un radical aliphatique insaturé qui, dans la mesure où il ne porte qu'une double liaison et où cette double liaison est en position $\alpha,\beta$- ou en position $\beta,\gamma$- par rapport à l'atome de carbone voisin de $R^1$ et

$R^2$, n'est pas ramifié en position $\beta$, ou dont l'atome de carbone en position $\gamma$ est encore relié à un hétéroatome ou à au moins un atome de carbone en position $\delta$; $R^1$ et $R^2$ étant également mis, avec l'atome de carbone voisin, pour des termes d'un radical alicyclique ou d'un éther cyclique, par oxydation d'alcools avec de l'oxygène en présence d'un catalyseur métallique, caractérisé en ce qu'on oxyde, à une température de 450 à 700° C, pendant une durée de séjour de 0,1 s au maximum, des alcools de formule II

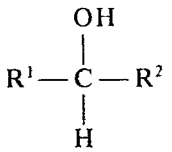

(II)

dans laquelle $R^1$ et $R^2$ ont les significations données ci-dessus, en présence de cristaux d'argent et/ou de cristaux de cuivre ayant une grosseur de grains comprise entre 0,01 microns et 2,5 mm, en tant que catalyseur.